# EUROPÄISCHE PATENTSCHRIFT

(11) **EP 1 566 151 B1**
(45) Veröffentlichungstag und Bekanntmachung des Hinweises auf die Patenterteilung: **12.10.2011**
(21) Anmeldenummer: 05001310.1
(22) Anmeldetag: 24.01.2005
(51) Int. Cl.: A61C 3/14, A61B 17/28

(54) **Dentalmedizinische Zange mit trennbaren Hälften**
Dental forceps with separable halves
Forceps dentaire composé de deux moitiés séparables

(30) Priorität: 19.02.2004 DE 202004002560 U
(43) Veröffentlichungstag der Anmeldung: 24.08.2005
(73) Patentinhaber: Helmut Zepf Medizintechnik GmbH, 78606 Seitingen-Oberflacht (DE)
(72) Erfinder: Zepf, Helmut, 78606 Seitingen-Oberflacht (DE)
(74) Vertreter: Patentanwälte Westphal, Mussgnug & Partner

(56) Entgegenhaltungen:
- DE-C- 743 853
- FR-A- 401 954
- US-A- 3 735 763
- US-A- 4 662 372

## Beschreibung

Die Erfindung betrifft eine dentalmedizinische Zange, insbesondere eine Extraktionszange .

Zangen allgemein und insbesondere dentalmedizinische Zangen, z. B. Extraktionszangen bestehen aus zwei Zangenteilen, die jeweils eine Backe und einen Griffschenkel aufweisen. Die Zangenteile sind durch ein Gelenk, auch Schloss oder Gewerbe genannt, schwenkbar miteinander verbunden. Die Backen der Zangenteile bilden das Zangenmaul, während die Griffschenkel den Zangengriff bilden. Bei den bekannten Zangen ist das Gelenk als aufgelegtes Gelenk, bei dem die beiden Zangenteile aufeinander gelegt und verbunden werden, als eingelegtes Gelenk, bei dem das eine Zangenteil in eine Ausfräsung des anderen Zangenteils eingelegt und mit diesem verbunden wird, oder als durchgestecktes Gelenk ausgebildet, bei welchem das eine Zangenteil durch eine Aussparung des anderen Zangenteils hindurchgeschoben wird. Die beiden Zangenteile werden in der Schwenkachse miteinander vernietet, so dass die beiden Zangenteile untrennbar miteinander verbunden sind. Ebenso ist es bekannt, die beiden Zangenteile durch eine in der Schwenkachse angeordnete Schraube miteinander zu verbinden. Hier ist eine Trennung der Zangenteile möglich, was insbesondere für die Reinigung, Desinfektion und gegebenenfalls Sterilisierung vorteilhaft ist. Das Zerlegen der Zange ist dabei jedoch umständlich und zeitraubend.

Das Dokument DE 743 853 C offenbart ein chirurgisches Instrument mit einem aus einem mit Umfangslappen versehenen Zapfen und einer entsprechenden Bohrung gebildeten lösbaren Gelenk. Der Erfindung liegt die Aufgabe zugrunde, eine dentalmedizinische Zange zu schaffen, die einfach zerlegt und wieder zusammengesetzt werden kann.

Diese Aufgabe wird erfindungsgemäß gelöst durch eine dentalmedizinische Zange mit den Merkmalen des Anspruchs 1.

Vorteilhafte Ausführungen der Erfindung sind in den rückbezogenen Unteransprüchen angegeben.

Bei der erfindungsgemäßen Zange wird das Gelenk durch einen an dem einen Zangenteil ausgebildeten Zapfen und eine in dem anderen Zangenteil ausgebildete entsprechende Bohrung gebildet. Der Zapfen wird in die Bohrung eingesteckt und bildet das Schwenkgelenk, bei welchem der kreiszylindrische Zapfen in der mit demselben Durchmesser ausgebildeten kreiszylindrischen Bohrung geführt wird. Die Bohrung weist in ihrer Wandung eine Umfangsnut auf und entsprechend weist der Zapfen an seiner Außenumfangsfläche wenigstens einen Umfangslappen auf. Der Umfangslappen des Zapfens greift in die Umfangsnut der Bohrung ein, wodurch der Zapfen axial formschlüssig in der Bohrung gehalten wird und wodurch die zwei Zangenteile schwenkbar miteinander verbunden sind. Um die Zange zerlegen und wieder zusammensetzen zu können, ist der wenigstens eine Umfangslappen nur über einen Teilwinkelbereich des Umfangs ausgebildet. Werden die zwei Zangenteile in ihrer gegenseitigen Winkelstellung so ausgerichtet, dass der wenigstens eine Umfangslappen im Winkelbereich mit zu der Umfangsnut führenden Eintrittsrinnen zur Deckung kommt, so kann der Zapfen in die Bohrung eingesteckt oder aus dieser herausgezogen werden. Diese Winkelstellung entspricht der Spreizstellung der Zange mit maximaler Öffnung des Zangenmaules. Ist der Zapfen in die Bohrung eingesteckt, so wird die Zange in ihre Schließ- bzw. Greifstellung verschwenkt. Dabei wird auch der Umfangslappen in seiner Winkelstellung gegen die Umfangsnut verschwenkt, so dass der Umfangslappen in die Umfangsnut eingreift und der Zapfen dadurch axial formschlüssig in der Bohrung gehalten wird. Während des Gebrauchs der Zange werden die Zangenteile entsprechend der erforderlichen Öffnung des Zangenmaules nur in einem Spreizwinkelbereich verschwenkt, in welchem der Umfangslappen nicht aus der Umfangsnut herausgedreht wird. Die beiden Zangenteile sind daher für den Gebrauch der Zange stets zuverlässig miteinander verbunden. Soll die Zange zur Reinigung zerlegt werden, so müssen die Zangenteile nur über die normale Gebrauchsschwenkstellung hinaus auseinander gespreizt werden, bis der wenigstens eine Umfangslappen in den Winkelbereich der wenigstens einen Eintrittsrinne kommt, so dass der Zapfen nun axial aus der Bohrung herausgezogen werden kann und die beiden Zangenteile voneinander getrennt werden können. Sowohl für das Zerlegen als auch für das Zusammensetzen der Zange sind somit keine zusätzlichen Verbindungsmittel erforderlich, die in umständlicher Weise befestigt bzw. gelöst werden müssen. Es genügt sowohl für das Zerlegen als auch für das Zusammensetzen ein einfacher Handgriff, ohne dass zusätzliche Werkzeuge erforderlich sind.

In einer vorteilhaften Ausführung ist die Bohrung als an ihrem Grund geschlossene Sackbohrung ausgebildet. Dadurch sind weder der Zapfen noch die Bohrung an der jeweiligen Gelenkaußenseite der Zangenteile sichtbar.

Weiter kann eine Bremseinlage zwischen den miteinander in Berührung stehenden Flächen des Zapfens und der Bohrung vorgesehen sein. Diese Bremseinlage hat die Funktion, einen definierten Widerstand bei der Schwenkbewegung der Zange zu bewirken. Durch die Wahl des Materials und der Dimensionierung der Bremseinlage kann ein definiert vorgegebener Widerstand erzeugt werden, so dass die Zange einerseits ausreichend leichtgängig betätigt werden kann und andererseits sich nicht unbeabsichtigt aus der jeweiligen Schwenkstellung bewegt. Ist die Bohrung als Sackbohrung ausgebildet, so kann die Bremseinlage zweckmäßig als Scheibe zwischen den Grund der Bohrung und die axiale Stirnfläche des Zapfens eingesetzt werden. Dadurch ist eine gleichmäßige Bremswirkung in jeder Drehstellung und ein gleichmäßiger Verschleiß der Bremseinlage gewährleistet.

Die Erfindung betrifft auch eine kostengünstige Fertigung der oben beschriebenen dentalmedizinischen Zange.

Bei bekannten dentalmedizinischen Zangen werden zunächst einstückige Rohlinge für die Zangenteile als Press-Schmiedeteile hergestellt, die dann bearbeitet werden. Diese Herstellung ist arbeitsintensiv und daher kostenaufwendig. Erfindungsgemäß werden die Zangenteile der oben beschriebenen Zange dagegen aus zwei Stücken gefertigt. Die Teile der Zange, die eine hohe Präzision erfordern, nämlich das Zangenmaul und das Gelenk werden gefräst bzw. gedreht und gebohrt oder durch Druckguss hergestellt, während die Griffschenkel an das jeweilige die Backe und das Gelenk bildende Stück des Griffteiles angesetzt werden. Für diese Griffschenkel sind weniger hohe Anforderungen in Bezug auf das Material und die Bearbeitungspräzision erforderlich. Dadurch ergibt sich insgesamt eine Ersparnis an Material- und Herstellungskosten. Außerdem ist es möglich, Zangenmäuler und Zangengriffe in unterschiedlicher Weise miteinander zu kombinieren, so dass eine größere Typen-Vielfalt der Zange hergestellt werden kann, ohne dass für jede Type ein eigener Rohling gefertigt werden muss.

Im folgenden wird die Erfindung anhand von in der Zeichnung dargestellten Ausführungsbeispielen näher erläutert. Es zeigen:
Figur 1 eine im Bereich des Gelenks axial geschnittene Seitenansicht der Zange in einer ersten Ausführung im zerlegten Zustand,
Figur 2 eine Seitenansicht des Gelenkbereichs des zweiten Zangenteils,
Figur 3 eine Draufsicht auf den Gelenkbereich des zweiten Zangenteils,
Figur 4 eine Seitenansicht des Gelenkbereichs des ersten Zangenteils,
Figur 5 eine Draufsicht auf den Gelenkbereich des ersten Zangenteils,
Figur 6 eine Seitenansicht des Gelenkbereichs der zusammengesetzten Zange,
Figur 7 eine Draufsicht auf den Gelenkbereich der zusammengesetzten Zange,
Figur 8 zwei Ansichten der zusammengesetzten Zange in der Ausführung der Figur 1,
Figur 9 zwei Ansichten der Zange in einer zweiten Ausführung,
Figur 10 zwei Ansichten der Zange in einer dritten Ausführung und
Figur 11 zwei Ansichten der Zange in einer vierten Ausführung.

Die Zange die im Ausführungsbeispiel als Zahn-Extraktionszange dargestellt ist, besteht aus zwei Zangenteilen 10 und 12. Die beiden Zangenteile 10 und 12 bestehen in an sich bekannter Weise aus jeweils einer Backe 14 bzw. 16, einem Griffschenkel 18 bzw. 20 und einem Gelenkbereich 22 bzw. 24, der die jeweilige Backe 14 bzw. 16 mit dem jeweiligen Griffschenkel 18 bzw. 20 verbindet. Die Backen 14 und 16 bilden das Zangenmaul und sind dem jeweiligen Anwendungszweck der Zange entsprechend ausgebildet. Die Griffschenkel 18 und 20 bilden den Zangengriff und sind ebenfalls entsprechend dem jeweiligen Verwendungszweck der Zange angepasst.

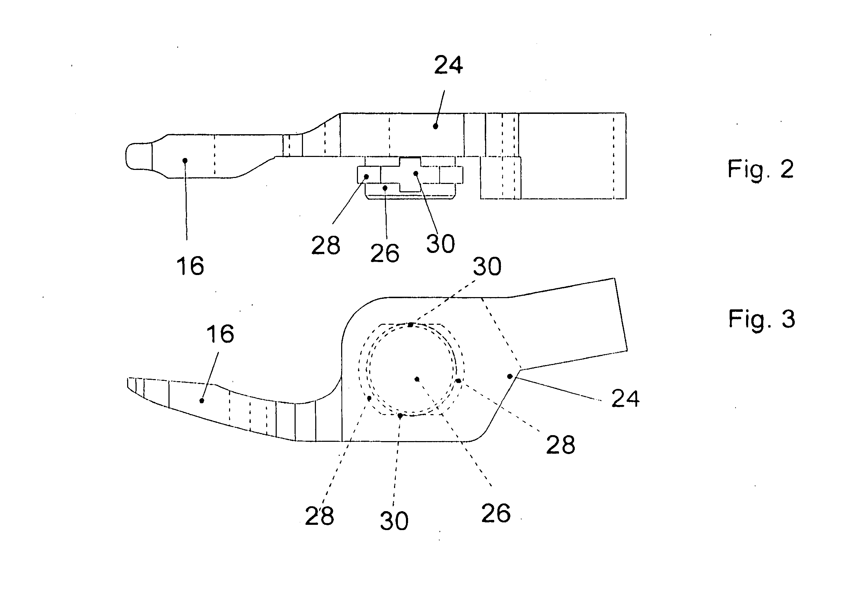

Die beiden Zangenteile 10 und 12 sind durch ein Gelenk schwenkbar miteinander verbunden, welches nachfolgend anhand der Figuren 2 bis 7 im Einzelnen erläutert wird. Im dargestellten Ausführungsbeispiel ist das Gelenk als eingelegtes Gelenk ausgebildet, bei welchem die Gelenkbereiche 22 und 24 jeweils eingefräste Aussparungen aufweisen, in die der korrespondierende Gelenkbereich 22 bzw. 24 des anderen Zangenteils 10 bzw. 12 eingelegt wird. Es ist offensichtlich, dass das Gelenk auch als aufgelegtes Gelenk ausgebildet sein kann.

In den Figuren 2 und 3 ist der Gelenkbereich 24 des zweiten Zangenteiles 12 dargestellt. An dem Gelenkbereich 24 ist ein gegen den Gelenkbereich 22 des ersten Zangenteils 10 gerichteter Zapfen 26 angeformt. Der Zapfen 26 hat die Form eines geraden Kreiszylinders, dessen Mittelachse mit der Schwenkachse der Zange zusammenfällt. Etwa auf halber Höhe des Zapfens 26 ist an dessen Außenumfang ein Umfangsbund 28 angeformt. Der Umfangsbund ist an zwei diametral liegenden Seiten abgefräst, so dass sich parallel zur Längserstreckung des Zangenteiles 12 verlaufende Flachseiten 30 ergeben. Die Abfräsung ist bis auf den Durchmesser des Zapfens 26 geführt, so dass die Flachseiten 30 einen Durchmesser des Zapfens 26 entsprechenden Abstand aufweisen. Der Umfangsbund bildet dadurch zwei diametral angeordneten Umfangslappen 28, die in Richtung der Längserstreckung des Zangenteils 12 in distaler bzw. proximaler Richtung abstehen. Die axiale Höhe des Zapfens 26 ist etwas geringer als die den Gelenkbereich 22 des ersten Zangenteils 10 aufnehmende Einfräsung des Gelenkbereichs 24, wie in Figur 2 durch eine strichpunktierte Linie angedeutet ist.

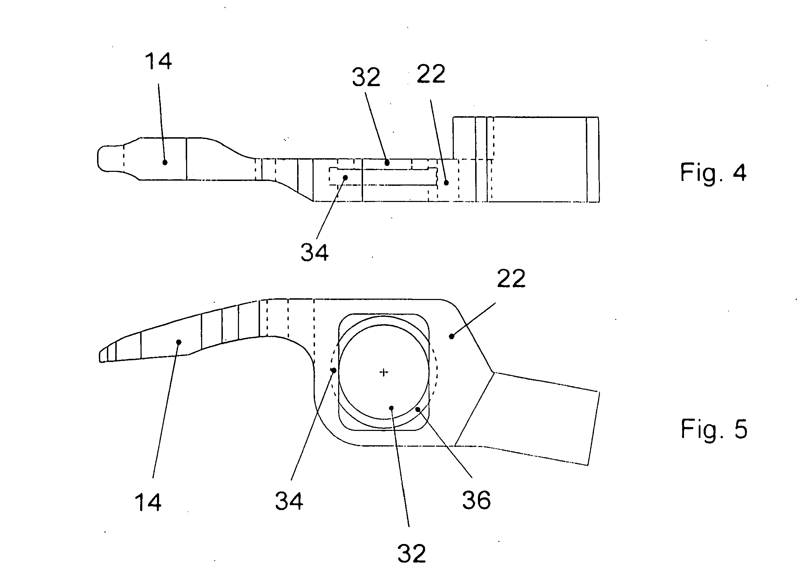

In den Figuren 4 und 5 ist der Gelenkbereich 22 des ersten Zangenteils 10 dargestellt. Dieser Gelenkbereich 22 weist eine Bohrung 32 auf, deren Durchmesser dem Durchmesser des Zapfens 26 entspricht und deren Mittelachse mit der Schwenkachse der Zange zusammenfällt. Die Bohrung 32 ist als Sackbohrung ausgebildet, deren axiale Tiefe geringfügig größer ist als die axiale Höhe des Zapfens 26. In halber Tiefe der Bohrung 32 ist in deren Innenumfang eine Umfangsnut 34 eingearbeitet. Die Umfangsnut 34 entspricht in ihrer axialen Position, in ihrer axialen Breite und in ihrer radialen Tiefe jeweils der axialen Position, der axialen Breite und der radialen Höhe der Umfangslappen 28 des Zapfens 26. Auf der dem zweiten Zangenteil 12 zugewandten Seite des Gelenkbereiches 22 ist der Durchmesser der Bohrung 32 auf zwei diametralen Seiten bis in die Umfangsnut 34 hinein auf den Aussendurchmesser der Umfangsnut 34 erweitert. Dadurch werden zwei Eintrittsrinnen 36 gebildet, die von der Oberfläche des Gelenkbereichs 22 bis in die Umfangsnut 34 führen und in der Querschnittsform und den Querschnittsabmessungen den Umfangslappen 28 des zweiten Zangenteils 12 entsprechen. Die Eintrittrinnen 36 sind so ausgerichtet, dass sie senkrecht zur Längserstreckung des Zangenteils 10 weisen. Um die beiden Zangenteile 10 und 12 zusammenzusetzen, werden die beiden Zangenteile 10 und 12 mit ihren Längsachsen senkrecht zueinander angeordnet. Dadurch kommen die Umfangslappen 28 in ihrer Winkelstellung mit den Eintrittsrinnen 36 zur Deckung. Der Zapfen 26 kann nun in die Bohrung 32 axial eingeschoben werden, wobei die Umfangslappen 28 durch die Eintrittrinnen 36 in den axialen Bereich der Umfangsnut 34 gelangt. Nun werden die Zangenteile 10 und 12 in Schließrichtung gegeneinander verschwenkt. Dadurch werden die Umfangslappen 28 gegenüber den Eintrittsrinnen 36 verdreht und treten in die Umfangsnut 34 ein. In dieser Stellung ist die Zange für den Einsatz bereit. Dabei ist der Zapfen 26 in der Bohrung 32 drehbar geführt und wird durch die in die Umfangsnut 34 eingreifenden Umfangslappen 28 in axialer Richtung formschlüssig in der Bohrung 32 gehalten.

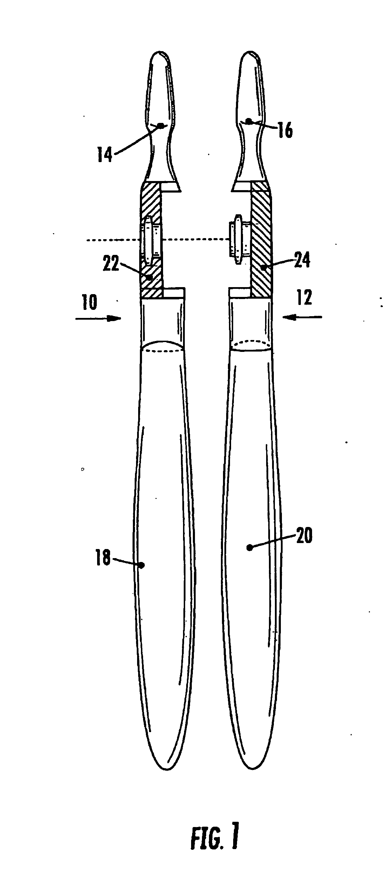

Es ist offensichtlich, dass die Bohrung 32 auch als durchgehende Bohrung ausgebildet sein kann, wie dies in Figur 1 dargestellt ist. Die Ausbildung der Bohrung 23 als Sackbohrung hat den Vorteil, dass das Gelenk weder von der Außenseite des Gelenkbereichs 22 noch von der Außenseite des Gelenkbereichs 24 sichtbar ist.

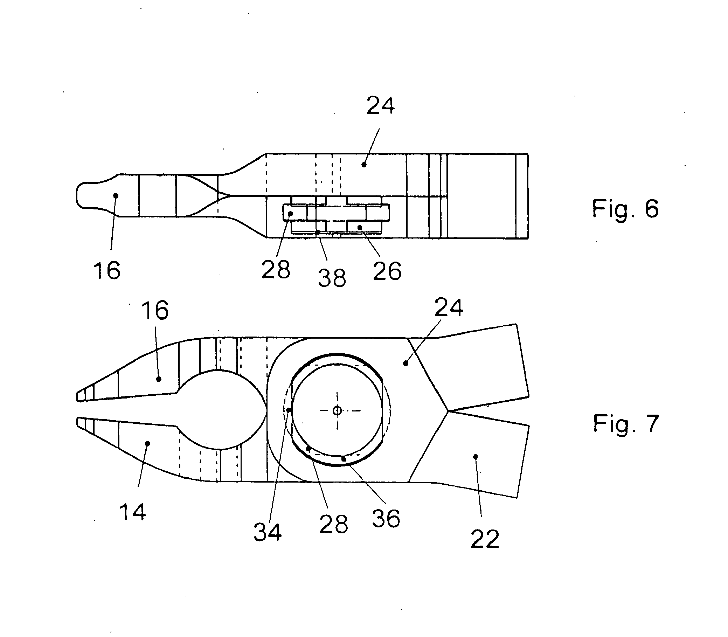

Um die Zange leichtgängig öffnen und schließen zu können und dennoch eine unbeabsichtigte Schwenkbewegung der Zangenteile 10 und 12 gegeneinander zu verhindern, ist vorzugsweise eine Bremseinlage 38 in das Gelenk eingesetzt. Die Bremseinlage 38 kann aus einem geeigneten Material z.B. einem Kunststoff bestehen, welches eine definierte Elastizität und Gleitfähigkeit in der Paarung mit dem Metall der Gelenkbereiche 22 bzw. 24 aufweist. Die Bremseinlage 38 kann beispielsweise in eine der axialen Stirnflächen der Umfangsnut 34 eingesetzt werden, so dass sie in Bremswirkung mit den Umfangslappen 28 des Zapfens tritt. Ist die Bohrung 32 als Sackbohrung ausgebildet, wie dies in den Figuren 2 bis 7 dargestellt ist, so wird die Bremseinlage 38 vorzugsweise als Kreisscheibe am Grund der Bohrung 32 angeordnet, so dass sie in Bremswirkung mit der axialen Stirnfläche des Zapfens 26 tritt ; wie dies in Figur 6 angedeutet ist. Durch die Wahl des Materials und der Stärke der Bremseinlage 38 kann die Leichtgängigkeit bzw. die Bremswirkung definiert vorgegeben werden.

Anstelle von zwei diametralen Umfangslappen 28 kann auch nur ein radial abstehender Umfangslappen vorgesehen sein, wobei entsprechend auch nur eine Eintrittsrinne 36 vorgesehen ist. Auch hier ist die Anordnung des Umfangslappens 28 und der Eintrittsrinne 36 so ausgerichtet, dass die Querschnittsfläche des Umfangslappen 28 mit der Querschnittsfläche der Eintrittsrinne 36 dann zur Deckung kommt, wenn die Zangenteile 10 und 12 entsprechend einer maximalen Öffnungsspreizung der Zange ausgerichtet sind. In jeder anderen Schwenkstellung greift der Umfangslappen 28 in die Umfangsnut 34 ein, so dass der Zapfen 26 axial formschlüssig in der Bohrung 32 gehalten wird und somit die Zangenteile 10 und 12 schwenkbar miteinander verbunden sind.

Die Backe 14 mit dem Gelenkbereich 22 und die Backe 16 mit dem Gelenkbereich 24 sind jeweils als einstückiges Teil aus einem geeigneten Metall, insbesondere einem geeigneten Stahl gefertigt. Diese Teile werden insbesondere durch Fräsen, Drehen und Bohren hergestellt. Das verwendete Material und die Herstellungsverfahren gewährleisten eine optimale Präzision und Festigkeit in den für die Verwendung der Zange wesentlichen Teilen, nämlich dem Zangenmaul und dem Gelenk.

Die Griffschenkel 18 und 20 werden als getrennte Werkstücke hergestellt. Die Griffschenkel 18 und 20 werden mit den jeweiligen Gelenkbereichen 22 bzw. 24 fest verbunden. Hierzu können die Griffschenkel 18 und 20 mit den Gelenkbereichen 22 bzw. 24 verzapft, verlötet oder verklebt werden. Da an den durch die Griffschenkel 18 und 20 gebildeten Zangengriff keine so hohen Anforderungen in Bezug auf die Präzision und die Materialeigenschaften gestellt werden müssen, kann die Auswahl des Materials und des Herstellungsverfahrens für die Griffschenkel 18 und 20 unter dem Gesichtspunkt der günstigsten Herstellungskosten getroffen werden.

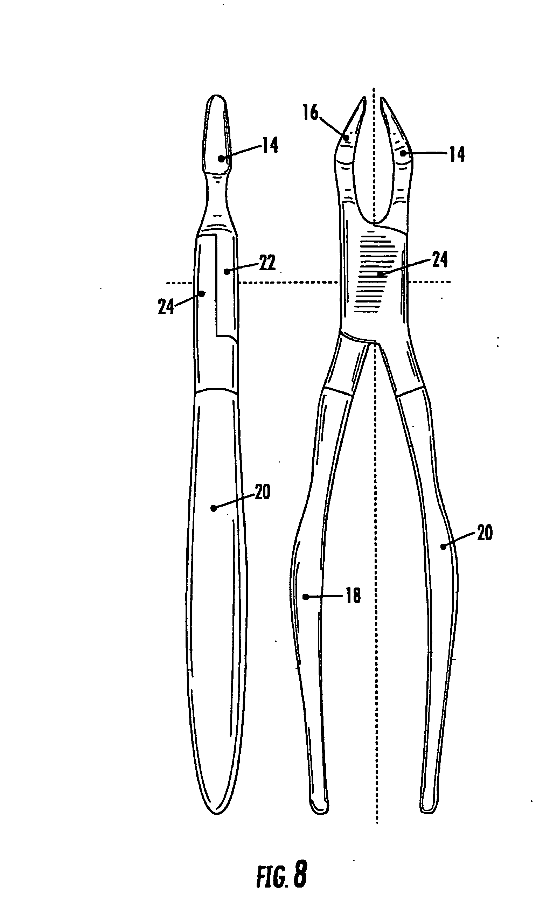

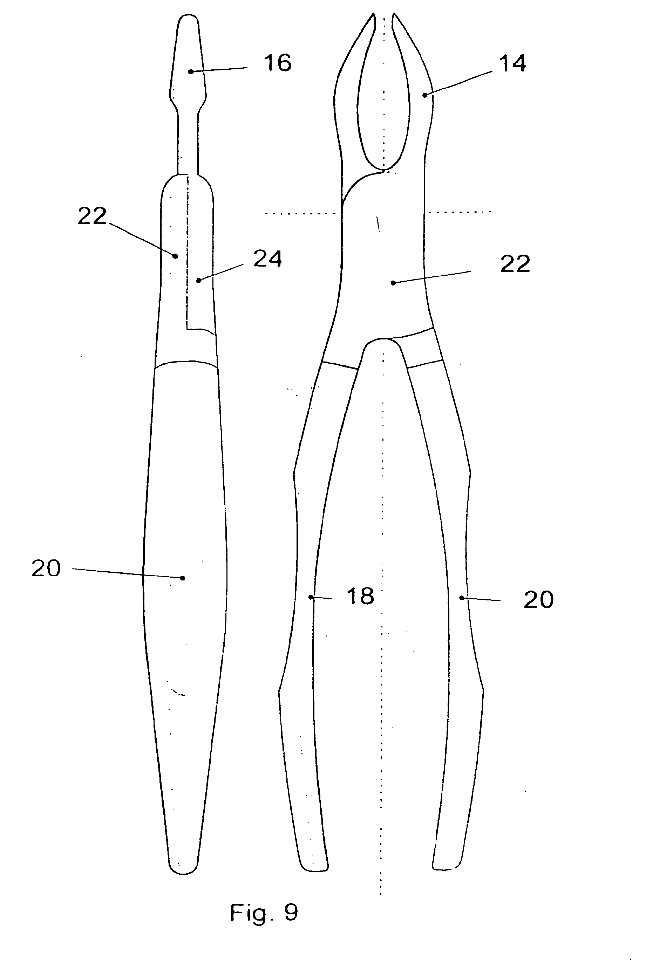

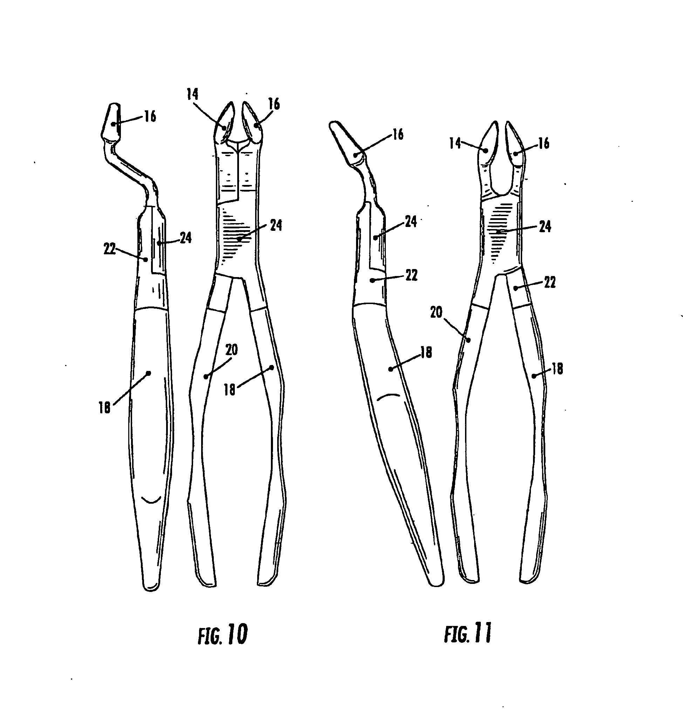

Weiter hat die Herstellung der Backen 14 bzw. 16 und der Gelenkbereiche 22 bzw. 24 einerseits und der Griffschenkel 18 bzw. 20 andererseits als getrennte Werkstücke den Vorteil, dass Zangenmaul und Griffform in unterschiedlicher Weise miteinander kombiniert werden können. Dies ist anhand der Figuren 8 bis 11 beispielsweise dargestellt. Figur 8 zeigt die Zange in der in Figur 1 dargestellten Ausführung. Figur 9 zeigt eine Zange mit demselben Zangenmaul, wie dies die Zange der Figur 8 aufweist, die Griffschenkel 18 und 20 sind jedoch unterschiedlich ausgebildet. Figur 10 zeigt eine Zange, mit einem abgekröpften Zangenmaul, welches mit Griffschenkeln 18 und 20 kombiniert ist, die den Griffschenkeln 18 und 20 der Ausführung der Figur 9 entsprechen. Figur 11 zeigt eine Zange mit einem abgewinkelten Zangenmaul und einer weiteren abgewinkelten Ausführung der Griffschenkel 18 und 20.

### Bezugszeichenliste

- 10: erstes Zangenteil
- 12: zweites Zangenteil
- 14: Backe von 10
- 16: Backe von 12
- 18: Griffschenkel von 10
- 20: Griffschenkel von 12
- 22: Gelenkbereich von 10
- 24: Gelenkbereich von 12
- 26: Zapfen
- 28: Umfangslappen
- 30: Flachseiten
- 32: Bohrung
- 34: Umfangsnut
- 36: Eintrittsrinnen
- 38: Bremseinlage

## Patentansprüche

1. Dentalmedizinische Zange mit zwei Zangenteilen, die jeweils eine Backe des Zangenmaules und einen Griffschenkel des Zangengriffes aufweisen und durch ein Gelenk schwenkbar miteinander verbunden sind, wobei das Gelenk einen zur Schwenkachse koaxialen kreiszylindrischen Zapfen (26) des einen Zangenteiles (12) aufweist, der in eine zur Schwenkachse koaxiale kreiszylindrische Bohrung (32) gleichen Durchmessers des anderen Zangenteils (10) eingreift und in dieser Bohrung (32) um die Schwenkachse drehbar ist, wobei der Zapfen (26) und die Bohrung (32) mit wenigstens einem Umfangslappen (28) und einer Umfangsnut (34) in axialer Richtung formschlüssig ineinandergreifen, wobei sich der wenigstens eine Umfangslappen (28) nur über einen Teilwinkelbereich des Umfangs erstreckt und wobei wenigstens eine der Querschnittsform des Umfangslappens (28) entsprechende Eintrittsrinne (36) axial zu der Umfangsnut (34) führt, wobei der wenigstens eine Umfangslappen (28) und die wenigstens eine Eintrittsrinne (36) in dem jeweiligen Gelenkbereich (22 bzw. 24) in ihrer Winkelanordnung so ausgerichtet sind, dass diese in der maximalen Öffnungsspreizstellung der Zangenteile (10, 12) miteinander zur Deckung kommen und der wenigstens eine Umfangslappen (28) durch die wenigstens eine Eintrittsrinne (36) in die Umfangsnut (34) eingeführt bzw. aus dieser herausgehoben werden kann, während in den anderen Spreizstellungen der Zangenteile (10, 12) der wenigstens eine Umfangslappen (28) in die Umfangsnut (34) eingreift und axial formschlüssig in dieser gehalten wird.

2. Zange nach Anspruch 1,
**dadurch gekennzeichnet, dass** das Gelenk als eingelegtes Gelenk ausgebildet ist.

3. Zange nach Anspruch 1 oder 2,
**dadurch gekennzeichnet, dass** der wenigstens eine Umfangslappen (28) an dem Zapfen (26) und die Umfangsnut (34) in der Bohrung (32) ausgebildet sind.

4. Zange nach einem der Ansprüche 1 bis 3,
**dadurch gekennzeichnet, dass** die Winkelstellungen des wenigstens einen Umfangslappens (28) und der wenigstens einen Eintrittsrinne (36) in den jeweiligen Gelenkbereichen (22) bzw. (24) so ausgerichtet sind, dass diese miteinander zur Deckung kommen, wenn die Zangenteile (10, 12) unter einem maximalen Öffnungsspreizwinkel von etwa 90° angeordnet sind.

5. Zange nach einem der Ansprüche 1 bis 4,
**dadurch gekennzeichnet, dass** zwei diametral zueinander angeordneten Umfangslappen (28) und zwei diametral zueinander angeordnete Eintrittsrinnen (36) vorgesehen sind.

6. Zange nach einem der Ansprüche 1 bis 5,
**dadurch gekennzeichnet, dass** die Bohrung (32) durchgehend ausgebildet ist.

7. Zange nach einem der Ansprüche 1 bis 5,
**dadurch gekennzeichnet, dass** die Bohrung (32) als eine an ihrem Grund geschlossene Sackbohrung ausgebildet ist.

8. Zange nach einem der Ansprüche 1 bis 7,
**dadurch gekennzeichnet, dass** zwischen der Wandung der Bohrung (32) und der Wandung des Zapfens (26) eine Bremseinlage (38) angeordnet ist.

9. Zange nach Anspruch 7 und 8,
**dadurch gekennzeichnet, dass** die Bremseinlage (38) zwischen dem Grund der Bohrung (32) und der axialen Stirnfläche des Zapfens (26) angeordnet ist.

10. Zange nach einem der Ansprüche 1 bis 9,
**dadurch gekennzeichnet, dass** die Zangenteile (10, 12) jeweils aus zwei Stücken gefertigt sind, nämlich aus einem den Griffschenkel (18 bzw. 20) bildenden Stück und einem den Gelenkbereich (22 bzw. 24) und die Backe (14 bzw. 16) bildenden Stück, und dass die zwei Stücke fest miteinander verbunden sind.

11. Zange nach Anspruch 10,
**dadurch gekennzeichnet, dass** die zwei Stücke miteinander verzapft, verschweißt oder verklebt sind.

12. Zange nach Anspruch 10 oder 11,
**dadurch gekennzeichnet, dass** das den Gelenkbereich (22 bzw. 24) und die Backe (14 bzw. 16) bildenden Stück durch Fräsen, Drehen und Bohren oder Druckguss hergestellt ist.

13. Zange nach einem der Ansprüche 10 bis 12,
**dadurch gekennzeichnet, dass** die zwei Stücke aus unterschiedlichen Materialien hergestellt sind.

## Claims

1. Dental forceps with two parts which in each case have a jaw of the forceps mouth and an arm of the forceps grip and are connected to each other in a pivotable manner by a joint, wherein the joint has a circular cylindrical pin (26) of one forceps part (12), the pin (26) being coaxial with the pivot axis and engaging in a circular cylindrical bore (32) - coaxial with the pivot axis and of the same diameter - of the other forceps part (10) and being rotatable in this bore (32) about the pivot axis, wherein the pin (26) and the bore (32) engage one in the other with at least one peripheral tab (28) and a peripheral groove (34) in the axial direction in a positively locking manner, wherein the at least one peripheral tab (28) extends only over a partial angular area of the periphery and wherein at least one inlet channel (36) corresponding to the cross-sectional shape of the peripheral tab (28) leads axially to the peripheral groove (34), wherein the at least one peripheral tab (28) and the at least one inlet channel (36) are orientated in their angular arrangement in the respective joint region (22 and 24 respectively) in such a way that they come into alignment with each other in the maximum spread-open position of the forceps parts (10, 12) and the at least one peripheral tab (28) can be introduced into the peripheral groove (34) or can be lifted out of it through the at least one entry channel (36), whilst in the other spreading positions of the forceps parts (10, 12) the at least one peripheral tab (28) engages in the peripheral groove (34) and is held in it axially in a positively locking manner.

2. Forceps according to claim 1, **characterized in that** the joint is designed in the form of an inlaid joint.

3. Forceps according to claim 1 or 2, **characterized in that** the at least one peripheral tab (28) is formed on the pin (26) and the peripheral groove (34) is formed in the bore (32).

4. Forceps according to any one of claims 1 to 3, **characterized in that** the angular positions of the at least one peripheral tab (28) and the at least one entry channel (36) are orientated in the respective joint regions (22) and (24) respectively, in such a way that they come into alignment with each other when the forceps parts (10, 12) are arranged at a maximum spread-open angle of approximately 90°.

5. Forceps according to any one of claims 1 to 4, **characterized in that** two peripheral tabs (28) arranged diametrically to each other and two entry channels (36) arranged diametrically to each other are provided.

6. Forceps according to any one of claims 1 to 5, **characterized in that** the bore (32) is made continuous.

7. Forceps according to any one of claims 1 to 5, **characterized in that** the bore (32) is designed in the form of a blind bore closed at its bottom.

8. Forceps according to any one of claims 1 to 7, **characterized in that** a brake inlay (38) is arranged between the wall of the bore (32) and the wall of the pin (26).

9. Forceps according to claims 7 and 8, **characterized in that** the brake inlay (38) is arranged between the bottom of the bore (32) and the axial front end of the pin (26).

10. Forceps according to any one of claims 1 to 9, **characterized in that** the forceps parts (10, 12) are produced in each case from two pieces, namely from a piece forming the gripping arm (18 and 20 respectively) and a piece forming the joint region (22 and 24 respectively) and the jaw (14 and 16 respectively), and the two pieces are connected to each other in a fixed manner.

11. Forceps according to claim 10, **characterized in that** the two pieces are pinned, welded or glued to each other.

12. Forceps according to claim 10 or 11, **characterized in that** the piece forming the joint region (22 and 24 respectively) and the jaw (14 and 16 respectively) is produced by milling, turning and boring or die-casting.

13. Forceps according to any one of claims 10 to 12, **characterized in that** the two pieces are produced from different materials.

## Revendications

1. Pince dentaire comportant deux parties de pinces ayant chacune un mors pour la boucle de la pince ainsi qu'une branche de préhension pour la poignée, les deux parties étant reliées l'une à l'autre, de manière articulée en pivotement,
* l'articulation comprend un goujon (26) cylindrique circulaire coaxial à l'axe de pivotement sur une partie de pince (12) qui pénètre dans un perçage cylindrique circulaire (32) coaxial à l'axe de pivotement, de même diamètre, réalisé dans l'autre partie de pince (10) et tourne dans ce perçage (32) autour de l'axe de pivotement,
* le goujon (26) et le perçage (32) ayant au moins une collerette périphérique (28) et une rainure périphérique de réception (34) s'interpénétrant par la forme dans la direction axiale,
* la collerette périphérique (28) n'occupe qu'une fraction angulaire de la périphérie, et
* au moins une gorge d'entrée (36) ayant une forme de section correspondant à celle de la collerette périphérique (28) conduit axialement à la rainure périphérique (34),
* la collerette périphérique (28) et la gorge périphérique (36) dans la zone d'articulation respective (22 ou 24) sont alignées dans leur disposition angulaire de façon que dans la position d'écartement en ouverture maximale des parties de pinces (10, 12), elles se chevauchent et que l'arête périphérique (28) s'introduise à travers une gorge d'entrée (36) dans la rainure périphérique (34) ou puisse être dégagée de celle-ci, alors que dans les autres positions d'écartement des parties de pinces (10, 12), la collerette périphérique (28) pénètre dans la rainure périphérique (34) et y est maintenue axialement par une liaison par la forme.

2. Pince selon la revendication 1,
**caractérisée en ce que**
l'articulation est une articulation intégrée.

3. Pince selon la revendication 1 ou 2,
**caractérisée en ce qu'**
au moins une collerette périphérique (28) est réalisée sur le goujon (26) et la rainure périphérique (34) est réalisée dans le perçage (32).

4. Pince selon l'une des revendications 1 à 3,
**caractérisée en ce que**
la position angulaire d'au moins une collerette périphérique (28) et celle d'au moins une gorge d'entrée (36) dans les zones d'articulation respectives (22, 24) sont alignées de façon à coïncider lorsque les parties de pinces (10, 12) sont mises dans un angle d'écartement d'ouverture maximum d'environ 90°.

5. Pince selon l'une des revendications 1 à 4,
**caractérisée par**
deux collerettes périphériques (28) diamétralement opposées et deux gorges d'entrée (36) diamétralement opposées.

6. Pince selon l'une des revendications 1 à 5,
**caractérisée en ce que**
le perçage (32) est traversant.

7. Pince selon l'une des revendications 1 à 5,
**caractérisée en ce que**
le perçage (32) est un perçage borgne avec un fond fermé.

8. Pince selon l'une des revendications 1 à 7,
**caractérisée par**
un insert de frein (38) interposé entre la paroi du perçage (32) et la paroi du goujon (26).

9. Pince selon les revendications 7 et 8,
**caractérisée en ce que**
l'insert de frein (38) est placé entre le fond du perçage (32) et la surface frontale axiale du goujon (26).

10. Pince selon l'une des revendications 1 à 9,
**caractérisée en ce que**
les parties de pinces (10, 12) sont réalisées respectivement en deux morceaux, à savoir une branche de poignée (18, 20) constituant l'un des morceaux et un morceau formant la zone d'articulation (22, 24) et le mors (14, 16), et **en ce que** les deux morceaux sont reliés solidairement l'un à l'autre.

11. Pince selon la revendication 10,
**caractérisée en ce que**
les deux morceaux sont réunis par un goujon, par une soudure ou par un collage.

12. Pince selon la revendication 10 ou 11,
**caractérisée en ce que**
le morceau formant la zone d'articulation (22, 24) et le mors (14, 16) est réalisé par fraisage, travail au tour, perçage ou injection.

13. Pince selon l'une des revendications 10 à 12,
**caractérisée en ce que**
les deux morceaux sont réalisés en des matériaux différents.
